# EUROPEAN PATENT SPECIFICATION

(11) **EP 4 181 768 B1**
(45) Date of publication and mention of the grant of the patent: **26.03.2025**
(21) Application number: 21739410.5
(22) Date of filing: 28.06.2021
(51) Int. Cl.: A61B 5/00, A61B 5/315, A61B 5/398, A61B 3/12, A61F 7/12, A61N 5/06, A61B 5/01, A61B 3/15, A61F 9/008

(54) **A DEVICE AND METHOD FOR OBTAINING ERG SIGNALS**
EINE VORRICHTUNG UND EIN VERFAHREN ZUR GEWINNUNG VON ERG-SIGNALEN
UN DISPOSITIF ET UNE MÉTHODE POUR OBTENIR DES SIGNAUX ERG

(30) Priority: 17.07.2020 FI 20205758
(43) Date of publication of application: 24.05.2023
(73) Proprietor: Maculaser Oy, 02150 Espoo (FI)
(72) Inventor: KAIKKONEN, Ossi, 02150 Espoo (FI); TURUNEN, Teemu, 02150 Espoo (FI); KOSKELAINEN, Ari, 00076 Aalto (FI)
(74) Representative: Berggren Oy
(86) International application number: PCT/FI2021/050498
(87) International publication number: WO 2022/013483

(56) References cited:
- WO-A1-2019/197726
- JP-B2- 6 586 597
- US-A- 5 382 987
- US-A1- 2010 253 910
- US-A1- 2018 289 265
- US-A1- 2019 159 673
- BACH MICHAEL ET AL: "The influence of ambient room lighting on the pattern electroretinogram (PERG)", DOCUMENTA OPHTHALMOLOGICA, 1 November 2002 (2002-11-01), Dordrecht, pages 281 - 289, XP055840372, Retrieved from the Internet <URL:http://citeseerx.ist.psu.edu/viewdoc/download?doi=10.1.1.571.169&rep=rep1&type=pdf> [retrieved on 20210913], DOI: 10.1023/A:1021254427782
- SRAMEK CHRISTOPHER ET AL: "Improved safety of retinal photocoagulation with a shaped beam and modulated pulse", PROCEEDINGS OF SPIE, vol. 7550, 8 February 2010 (2010-02-08), 1000 20th St. Bellingham WA 98225-6705 USA, XP055856256, ISSN: 0277-786X, ISBN: 978-1-5106-4548-6, DOI: 10.1117/12.841079
- PALLASAHO SATU: "Master's Programme in Life Science Technologies Retinal Pigment Epithelium Heating Treatment for Age-Related Macular Degeneration", 29 July 2019 (2019-07-29), XP055856183, Retrieved from the Internet <URL:https://aaltodoc.aalto.fi/bitstream/handle/123456789/39906/master_Pallasaho_Satu_2019.pdf?sequence=1&isAllowed=y> [retrieved on 20211029]
- PITKÄNEN MARJA ET AL: "In vivo monitoring of mouse retinal temperature by ERG photoresponses", EXPERIMENTAL EYE RESEARCH, ACADEMIC PRESS LTD, LONDON, vol. 187, 22 May 2019 (2019-05-22), XP085831697, ISSN: 0014-4835, [retrieved on 20190522], DOI: 10.1016/J.EXER.2019.05.015
- POKORNY JOEL ET AL: "Photostimulator allowing independent control of rods and the three cone types", VISUAL NEUROSCIENCE., vol. 21, no. 3, 1 May 2004 (2004-05-01), GB, pages 263 - 267, XP055839800, ISSN: 0952-5238, Retrieved from the Internet <URL:https://citeseerx.ist.psu.edu/viewdoc/download?doi=10.1.1.591.101&rep=rep1&type=pdf> [retrieved on 20210910], DOI: 10.1017/S0952523804213207

## Description

### TECHNICAL FIELD OF THE INVENTION

The invention relates to electroretinography (ERG) in general. More specifically, the invention relates to obtaining enhanced focal ERG signals.

### BACKGROUND OF THE INVENTION

Electroretinography (ERG) is a method where electrical signals (electrical response) of the retina are recorded during exposure of the retina to stimulus, such as light flashes, which may be useful in various cases, such as in diagnosis of retinal diseases.

In prior art devices for obtaining focal ERG signals, the obtained focal ERG signals are often a combined signal, comprising response signals from a stimulated area target area of the retina and disturbance response signals from an area outside of the stimulated area due to scattering of the stimulus light to nearby areas of the retina outside of the target area.

Known devices for obtaining ERG signals suffer from the disadvantage that the obtained ERG signals are insufficiently indicative of the state of the signaling of retinal neurons in the target area.

ERG may be used on its own, while it has also been discovered that ERG signals obtained during retinal heating (e.g. photothermal retinal therapies) could be used to determine the temperature of the retina, due to the temperature-dependency of the electrical signalling of the retina. An obtained ERG signal during retinal heating can therefore be indicative of a temperature (or at least a difference in temperature) that is occurring at the retina due to heating, e.g. laser heating. The temperature determination related to heating could then be used e.g. for controlling the retinal heating so that the retinal temperature is kept at a desired level.

Due to the ERG signals not being sufficiently indicative of local retinal signaling and the light adaptation caused by a treatment laser, also the retinal temperature determined from the signals may suffer from inaccuracy.

In retinal heating applications, a laser spot may be used to heat the retinal tissue. A laser spot comprising a top-hat profile is known to be used in the prior art, where irradiance of the laser spot is approximately constant within a circular target area, dropping to zero outside of it.

US20100253910 discloses an apparatus for ocular light stimulation where infrared light is provided via a ring slit and stimulus light and background light is additionally provided. Background light is projected onto the ocular fundus so as to spread in all directions about the center of the anterior ocular segment.

Document WO2019197726 presents a device for heating therapy of the retinal pigment epithelium. Laser beams are combined with beam splitters and each laser channel has its own adjustable optics before the beam splitter. Locations and sizes of laser spots are adjusted independently once the beams reach the retina.

Sramek Christopher et al "Improved safety of retinal photocoagulation with a shaped beam and modulated pulse", Proceedings of SPIE, vol. 7550, 8 February 2010 presents a ring-shaped beam for retinal photocoagulation for reducing maximum temperature in the center of the spot.

### SUMMARY OF THE INVENTION

The invention is set out in the appended set of claims.

### SUMMARY OF THE DISCLOSURE

The object of the disclosure is to alleviate at least some of the problems in the prior art. In accordance with one aspect of the present disclosure a device for obtaining retinal ERG signals from a target area of the retina is provided, the device comprising means for obtaining an electrical response signal from the target area and at least one light source configured to provide at least a stimulus beam configured to illuminate the target area for inducing an ERG signal and a light adapting background beam configured to illuminate the retina at least in an area outside of the target area, for light adapting the area outside of the target area and suppressing ERG signaling therefrom.

In one other aspect, the disclosure provides a device for heating at least a target area of the retina, the device comprising at least a heating light source for providing a heating beam to heat at least the target area, wherein the heating beam comprises an irradiance profile comprising an area of lower irradiance, where the brightness of the heating spot is lower than at an area of higher irradiance.

Having regard to the utility of embodiments of the disclosure, devices and methods for obtaining ERG signals are provided, where the obtained ERG signal may be enhanced in the sense that the obtained signal may be more accurately indicative of a response of the stimulated/target tissue (and not surrounding tissue).

The present disclosure may provide enhancements to accuracy of obtained ERG signals when ERG methods are used on their own or in cases where ERG is utilized during retinal heating, wherein the obtained ERG signal may be more effectively used to determine a retinal temperature.

The inventors have discovered that in prior art devices attempting to record ERG signals during retinal heating, the obtained results have also here been erroneous due to the origin of the obtained ERG signal. The obtained signal has not only been indicative of the ERG signal of a treatment/target/heated area of the retina, but also ERG signaling arising from surrounding areas of the retina have been comprised in the obtained signal due to scattering of the stimulus light to nearby areas of the retina outside of the target area. Thereby any further analysis based on the signal (such as determined retinal temperature) has not been accurate.

The inventors have also realized that in previous methods where retinal heating is utilized, the illumination of the fundus has been insufficient, leading to a heating laser affecting the obtained ERG signals through both light adaptation and rising of temperature of the retina, and these effects have not been distinguishable from each other. Also here, any further analysis based on the ERG signal has not been accurate.

In embodiments of the disclosure an obtained ERG signal may be more accurately indicative of the response of a target area of the retina to the stimulus by a stimulus beam, because a light adapting background beam may suppress the ERG signaling of an area of the retina outside of the target area by illuminating (and thereby light adapting) at least an area of the retina outside of the target area, reducing the ERG response elicited by scattered stimulus light. Also any further analysis involving the ERG signal may thus be more accurate.

In one embodiment of the disclosure a central background beam is bright enough so that the light adaptation effect of a heating laser is essentially insignificant. Therefore, obtained signals are only affected by the temperature elevation caused by the heating laser, and not by the changing light adaptation state. In one embodiment, the central background beam has a brightness of over 50 lux, advantageously over 100 lux at the fundus so that a heating beam does not to cause significant light adaptation of the target area.

During retinal heating, the fundus is usually imaged so that e.g. a physician conducting the retinal heating can monitor the eye and/or appropriately focus the heating, which is usually directed towards a small portion of the retina. Embodiments of the disclosure providing a light adapting background beam may provide enhanced ERG signals, but is was noticed by the inventors that the light adapting background beam may disturb the imaging of the fundus through backreflection artefacts. Therefore, one embodiment of the disclosure provides a light adapting background beam that is essentially blocked from fundus imaging using e.g. an optical notch filter blocking the light adapting background beam from imaging. Here, the imaging of the fundus may be carried out effectively without being disturbed by the light adapting background beam, due to disturbing reflections being blocked from reaching the imaging system, these disturbing reflections being reflections of the light adapting background beam e.g. from the surface of the fundus lens, from the surface of the eye, and/or scattered from inside the eye.

In one embodiment, the light adapting background beam comprises polarized light and the light adapting background beam is blocked from fundus imaging using a polarizer.

In one more embodiment, the light adapting background beam is modulated with an on/off waveform and a camera sensor of an imaging module is synchronized to be exposed essentially only when the light adapting background beam is off to prevent the light adapting background beam from reaching the imaging system.

In one embodiment involving retinal heating, the stimulus beam may be of equal size or smaller than a heating beam used for heating a heating area, which corresponds to at least the target area when directed at a final location with relation to the retina, optionally the stimulus beam may have a beam area such that when directed at a final location with relation to the retina, the stimulus beam area is around 50-90%, preferably about 70-80% of the heating beam area (e.g. diameter and concentric to the heating beam/spot). The rise in temperature of the retinal tissue due to the heating beam may be highest at the center of the heating beam area and falls towards the edges of the heating beam. It is beneficial to induce an ERG signal of the retinal response with the stimulus beam specifically at the center of the heating beam (and therefore at the center of the target area), so that the ERG signal is indicative of a temperature of the target area that is subject to the highest rise in temperature.

In one embodiment, the stimulus beam may be modulated with impulse-like flashes of light. In another embodiment, the stimulus may be a square wave. In a third embodiment the stimulus beam may be modulated with white noise.

The light adapting background beam may be adapted to illuminate an area on the eye from essentially at or near the border of the target area to or beyond the equator of the eye. A light adapting background beam that essentially illuminates an area of the retina that reaches from essentially at or near the border of the target area, said border being a border defined by an outer perimeter of a heating area, to the equator of the eye or beyond may more effectively suppress/prevent ERG signaling that could arise from areas of the eye outside of the target area.

The light adapting background beam may comprise an area of lower or no illumination/irradiation corresponding to the stimulus beam such that when the stimulus beam and the light adapting background beam are directed at a final location with respect to an eye, the area of lower or no illumination essentially overlaps with the stimulus beam. The area of lower or no illumination may thus essentially correspond to the target area. The area of lower illumination may e.g. comprise an irradiance that is at least 50% lower than that of the light adapting background beam at other areas, the irradiance may also be preferably e.g. at least 70% lower or more preferably at least 90% lower. Such a "dark spot" at e.g. the center of the light adapting background beam allows the target area to be at lower light adaptation level with respect to the periphery, and thus light scattering artefacts in the obtained ERG signal may be reduced as the target area is more sensitive to light than the periphery.

In yet one further aspect, the invention also relates to a device for heating a heating area or target area of the retina, the device comprising a heating light source that is configured to provide a heating beam that comprises an irradiance profile providing a heating spot comprising an area of lower irradiance essentially at the center of the heating spot, where the irradiance is lower than an irradiance at an area of higher irradiance at the edge of the heating spot.

An area of lower irradiance (such as no irradiance), may preferably be provided at the center of the heating beam, such that the heating beam (or at least the portion of the heating beam that has higher illuminance than a central spot) is annularly shaped.

It was considered by the inventors that the laser spots in the prior art with top-hat profile may be disadvantageous at least in connection with long-pulse retinal laser treatments, as the temperature elevation of the retina is highest at the center of the heating beam and falls towards the edges of the target area. It was recognized that a more even heat distribution provided by the heating beam may be advantageous in cases where a uniform treatment, i.e. uniform elevation of retinal tissue, is desired within the target area.

The area of lower illumination may e.g. comprise an irradiance that is at least 50% lower than that of the heating beam at other areas, the irradiance may also be preferably e.g. at least 70% lower or more preferably at least 90% lower. With this type of heating beam, a temperature profile of the heating beam may be more uniform and/or a lower temperature elevation may be provided at the center of the heated area. The annularly shaped heating spot may also be utilized in a device that obtains ERG signals during the retinal heating, also a device that also provides a light adapting background beam, such as the device described elsewhere in the present application. The annularly shaped heating spot may, however, be utilized in any retinal heating application.

The irradiance may increase essentially linearly or parabolically, for instance, between a first, lower irradiance value at the center of the heating spot and a second, higher irradiance value at the edge of the heating spot.

The irradiance may be 5-100% higher, advantageously 20-50% higher, at the edge of the heating spot compared to the irradiance at the center of the heating spot.

The area with lower irradiance may be substantially defined by a circle with a diameter from 0.1-1 mm, and may essentially be provided at the center of the heating spot.

In some embodiments, an irradiance profile may be selected based on a determined temperature increase of retinal tissue at the target area, optionally by determining a difference between temperature increase at the edge of the target area and temperature increase at the center of the target area. Heating of a target area refers herein, in embodiments involving both retinal heating and ERG stimulation, to heating of at least a target area that is illuminated by a stimulus beam. The heating may be delivered also to an area that is e.g. larger that the target area that is illuminated by the stimulus beam. The exemplary embodiments presented in this text are not to be interpreted to pose limitations to the applicability of the appended claims. The verb "to comprise" is used in this text as an open limitation that does not exclude the existence of unrecited features.

The novel features which are considered as characteristic of the invention are set forth in particular in the appended claims.

### BRIEF DESCRIPTION OF THE DRAWINGS

Next the invention will be described in greater detail with reference to exemplary embodiments in accordance with the accompanying drawings, in which:
Figure 1 shows one exemplary device according to an embodiment of the invention,
Figure 2 illustrates temperature increases at the retina using a heating beam with a traditionally shaped heating spot and annularly shaped heating spots with two different sizes of area of lower or no irradiation at the center of the heating spots, and
Figure 3 schematically depicts one exemplary device for retinal heating according to an embodiment of the invention.

### DETAILED DESCRIPTION

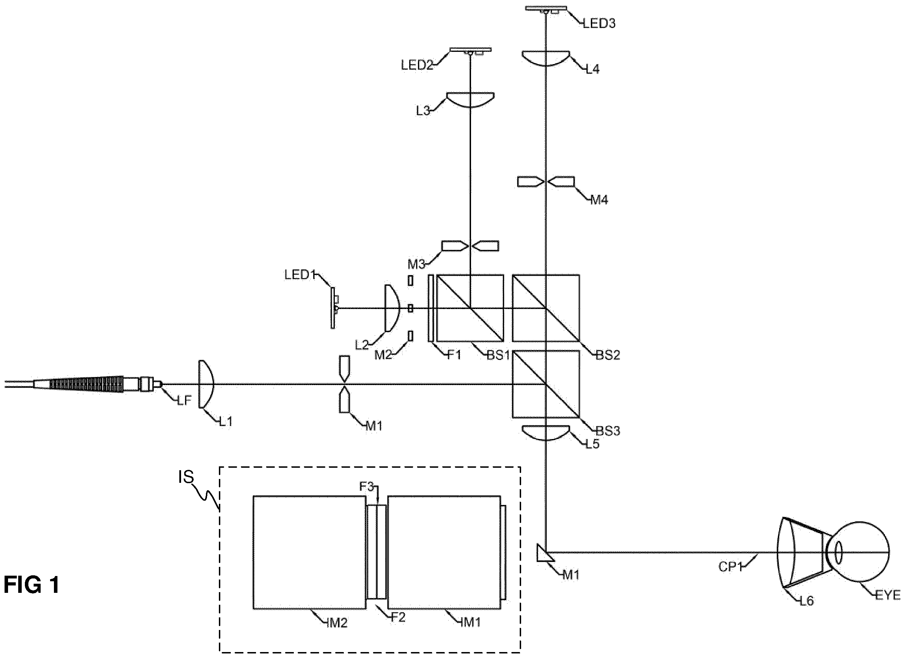

Figure 1 shows a device for obtaining retinal ERG signals according to one embodiment of the invention. The device is shown in Fig. 1 in connection with a retinal heating system and imaging system. The device comprises at least one light source. The at least one light source is configured to provide at least a stimulus beam for illuminating a target area of the retina to stimulate the target area of the retina to elicit a focal ERG signal. The device comprises at least one light source that is configured to provide a light adapting background beam for illuminating the retina at least in an area outside of the target area. A central background beam for illuminating at least the target area of the retina is may provided in the invention.

The provided beams may be generated using separate light sources, while e.g. the light adapting background beam and central background light beam may be provided by the same light source in one embodiment. The light sources are preferably controllable separately.

In the embodiment of Fig. 1, the stimulus beam is provided by a stimulus light source LED3. The stimulus beam is used to illuminate at least a target area of the retina, where the target area is an area from which ERG signals are to be obtained and optionally also an area which is heated/to be heated during retinal heating. The target area which is stimulated/illuminated with the stimulus beam in cases where retinal heating is not conducted may refer directly to the area that is illuminated by the stimulus beam, while in an embodiment where retinal heating is conducted, the target area may refer to the area that is heated, while the area illuminated by the stimulus beam in this case may not correspond to the entire target area. The stimulus beam is used to elicit/induce an ERG signal from the target area. The stimulus beam light source LED3 may be a light-emitting diode (LED) light source configured to provide a stimulus beam having wavelength of 500 - 600 nm, e.g. about 555 nm. Red and green cone cells have similar sensitivity at a wavelength of 555 nm, therefore a stimulus beam exhibiting a wavelength close to this may stimulate both cell types similarly. The stimulus beam may comprise white light, which may stimulate all retinal cone cells equally.

In one embodiment where retinal heating is conducted, the stimulus beam may be of equal size or smaller than a heating beam used for heating at least the target area. The stimulus beam may e.g. have a beam/spot diameter that is 50-90%, such as about 75% of a treatment beam diameter.

The stimulus light source LED3 may be configured to provide a stimulus light beam that is modulated. The stimulus beam therefore may not be provided as a continuous beam of light, but may comprise sequences, such as impulse-like flashes of light, pseudorandom waveforms or square waves. The modulation may be implemented e.g. at a frequency between 4 and 40 Hz, advantageously between 10 and 25 Hz.

Fig. 1 shows a light adapting background beam that is provided by a light adapting background light source LED1. The light adapting background beam is configured to illuminate the retina at least in an area outside of the target area, being configured to suppress or minimize ERG signaling from the area of the retina outside of the target area by light adapting the retina at least at the area outside of the target area. The light adapting background beam is advantageous in embodiments of devices where retinal heating is provided but also in devices configured to obtain ERG signals without retinal heating.

The light adapting background beam may comprise an area of lower or no illumination corresponding to the stimulus beam such that when the stimulus beam and the light adapting background beam are directed at a final location with respect to an eye, the area of lower or no illumination essentially overlaps with the stimulus beam. The target area (being an area illuminated by the stimulus beam and/or an area that is heated by a heating beam) may then not be illuminated by the light adapting background beam (or at least illuminated less than surrounding areas). The area of no illumination may e.g. correspond to a circular area with a diameter of 3 mm (for instance in systems where a stimulus beam has a diameter of 3 mm).

The light adapting background light source LED1 may be an LED light source. the light adapting background light source LED1 may also comprise a bandpass filter. The bandwidth of the light adapting background beam may be narrowed to for instance 10 nm.

The device of Fig. 1 is shown in connection with a fundus imaging system IS (which may in some embodiments also be realized as part of a device). The fundus imaging system IS may for instance be a biomicroscope or fundus camera or a scanning laser ophthalmoscope. The fundus imaging system may comprise a first imaging module IM1 and second imaging module IM2 and one or more filters, such as a second filter F2 and third filter F3.

The light adapting background beam may cause significant imaging artefacts due to backreflections as it reflects from surfaces on the beam path, such as from the fundus lens and the surface of the eye, back into the imaging optics. Therefore, it may be advantageous to configure the device such that the backreflections are essentially removed from retinal imaging or at least reduced. This can be achieved in numerous ways where the light adapting background beam is essentially prevented from reaching the imaging system.

The light adapting background beam may in one embodiment be blocked from fundus imaging using an optical filter blocking the light adapting background beam from imaging by a fundus imaging system. The optical filter may be an optical notch filter (band stop filter) and may be the second filter F2 of Fig. 1.

In one embodiment, the light adapting background beam may be blocked from fundus imaging by using polarized light to produce the light adapting background beam and using a polarizer blocking the light adapting background beam from imaging. The optical polarizer may be the second filter F2 shown in Fig. 1.

In one more embodiment, the backreflections may be removed from imaging by modulating the light adapting background beam with a fast on/off waveform, and synchronizing the imaging module camera exposure to only expose the camera sensor when the light adapting background beam is in an off-position.

In the device of Fig. 1, a central background light beam is provided by a central background light source LED 2. The central background light beam is configured to illuminate at least the target area of the retina and maintain a light adaptation level of the target area. The central background light beam may be concentric to the target area and may be limited to essentially illuminating the target area only. The central background light source LED 2 may be an LED light source. The central background light beam may comprise white light and/or may have a brightness of >100 lux at the fundus.

In other embodiments, the central background light beam may be provided by the same light source that is used to provide the light adapting background beam or the central background light beam may be provided by the same light source that is used to provide the stimulus light beam. For instance, the stimulus light source LED3 may be configured to provide a stimulus light beam where the light beam is maintained with a lower intensity between consecutive stimulating light pulses to provide the central background light beam between the stimulus pulses.

When used in connection with retinal heating, the brightness of the central background beam may be configured to be reduced as a heating laser or other heating means is turned on, to maintain a steady illuminance at the target area.

In embodiments of the invention where e.g. infrared imaging is used for fundus imaging, the central background light beam may not be needed.

A device may also comprise means for obtaining an ERG signal, i.e. means for obtaining a response signal of the target area to the stimulus provided by the stimulus beam. The ERG signal may be an electrical response that may be recorded/collected or obtained by one or more ERG electrodes. The electrodes may comprise one or more ocular electrodes and one or more reference electrodes. The ERG signal may be obtained as a voltage change between at least two electrodes over time.

The device may comprise or be usable in connection with a fundus lens L6. The fundus lens L6 may direct the provided light beams to the fundus of the eye. The fundus lens L6 may for instance be an inverting fundus lens with a field of view >120 degrees.

In one embodiment, a fundus lens may be integrated into the device, whereby a lens is not required to be placed on the cornea.

In one embodiment, a device comprises a fundus lens and ocular electrodes may be integrated into the fundus lens.

A heating system that the device may be usable with or which may also be part of the device may comprise at least a heat source, such as heating laser LF that is configured to elevate the temperature of the target area on the fundus. A heating light source LF may be configured to provide a heating beam that is directed to the target area.

The heating beam may comprise wavelength in the near-IR area. Wavelengths of light comprised in the heating beam may be 700-1000 nm, while the heating beam may be provided by a heating light source LF that is a fiber coupled diode laser.

In one embodiment, a heating beam has a homogenous irradiance profile and spot diameter of 1-6 mm on the fundus.

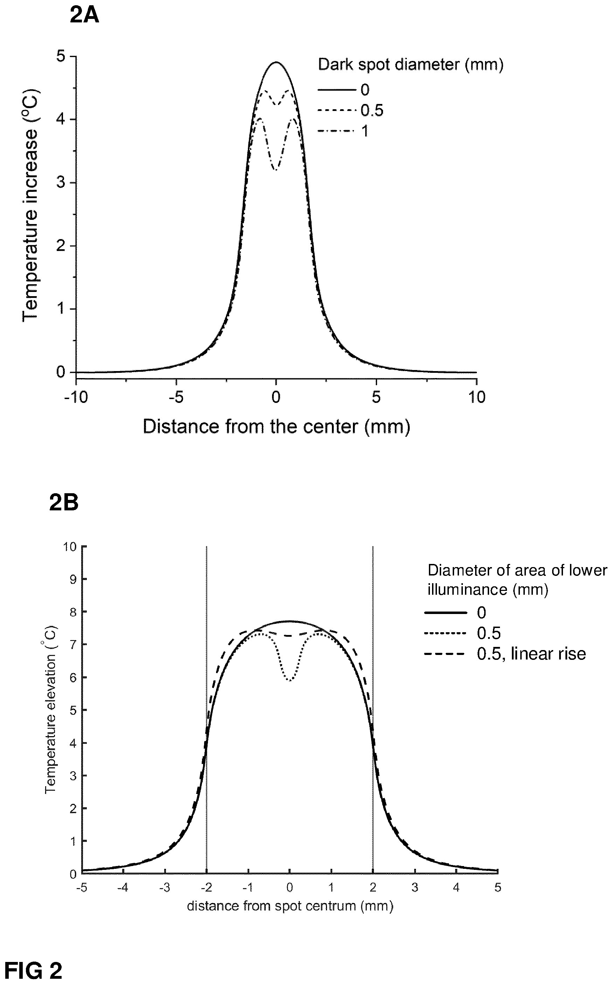

One embodiment of the invention also provides a device for retinal heating comprising a heating light source that is configured to provide a heating beam comprising an area of lower irradiance (such as no irradiance) at the center of the heating beam, such that the heating beam (or at least the portion of the heating beam that has higher illuminance than a central spot) is annularly shaped. The annularly shaped heating spot is advantageous with devices that obtain ERG signals during retinal heating, but may also be used to provide an improved retinal heating in cases where ERG stimulus is not provided. The annular shaped heating spot is discussed in more detail in connection with Figure 2.

In one embodiment, a heating beam may comprise or be associated with an aiming beam having essentially equivalent beam/spot size and irradiance profile as the heating beam. Optionally, a power of the aiming beam may be configured to be reduced when the heating beam is turned on to maintain an essentially steady illuminance at the target area.

Also other heating systems or means for heating may be utilized in connection with devices that are associated with retinal heating. For instance, the heating system may be implemented through ultrasound.

Considering the functioning of the device and associated other parts shown in Fig. 1, one use case scenario is described next. The four beams discussed above may be brought onto the same channel and projected onto the conjugate plane CP1. Four light channels may thus be involved, corresponding to the stimulus beam, light adapting background beam, central background light beam, and heating beam. The heating beam may be directed onto a first mask M1 by a first lens L1. The light adapting background beam may be directed by a second lens L2 onto a second mask M2. The central background light beam may be directed onto a third mask M3 by a third lens L3. A stimulus beam may be directed onto a fourth mask M4 by a fourth lens L4.

The fundus lens L6 may project the light profile at CP1 onto the fundus. The first imaging module IM1 is configured such that CP1 is either projected onto a camera sensor, or that the eyes of the treating physician are able to focus onto CP1 through biomicroscope eyepieces. The beams passed through a fifth lens L5 are directed towards the eye by a first mirror M1. The first mirror M1 may be placed directly in front of the first optics module IM1 (such as biomicroscope), so that the left eye has a view to the fundus on the left side of the first mirror M1, and the right eye has a view to the fundus on the right side of the first mirror M1. The fifth lens L5 may project images from masks M1, M2, M3 and M4 onto the conjugate plane CP1, meaning that the light profile emitted through the masks are imaged onto CP1. Beam splitters BS1, BS2 and BS3 may combine beams arising from the heating laser fiber output LF, and light sources LED1, LED2 and LED3.

In Fig. 1 the masks M1, M2, M3, M4 are holes and a light profile transmitted through the hole is projected onwards to the conjugate plane CP1. The masks may also be shaped mirrors or digital micromirror devices, in which case the light is made to reflect from the mask instead of passing through it. The masks may in some embodiments be used to achieve any dark spots or areas of lower or no irradiance in the light adapting background beam and/or the heating beam.

A first filter F1 may be used to make the spectrum of the light adapting background beam, provided by the light adapting background light source LED1, narrower. The passband of F1 may be 10 nm. The second filter F2 may be an optical notch filter with a 25 nm stop band centered at 530 nm, and may be used to block the light adapting background beam from entering the second imaging module IM2. The third filter F3 may be an infrared-cut-filter, which blocks laser light from being directed to imaging module IM2. The second imaging module IM2 may comprise a beams splitter which splits the imaging light to two channels and a camera system for one of the light channels and eye pieces for the other.

In some embodiments involving retinal heating, it may be beneficial for one part of the treated area to be in a lower temperature relative to the periphery. This may be the case when it is desirable to deliver a lower thermal dose to a specific area of the retina, e.g. the fovea. In these embodiments the irradiance profile may be designed to produce a lower temperature elevation in the center compared to the periphery. This may be achieved by having an area with a lower irradiance at the location of the heating spot that corresponds to a location of the target area of the retina where a lesser thermal dose is to be delivered. The area of lower irradiance may, for instance, be a circular 0.5 mm diameter area with essentially zero irradiance at the center of the laser spot at the fundus.

Figure 2 illustrates temperature increases at the retina in °C using a heating beam with a traditionally shaped top hat heating spot (solid trace, 0 mm diameter of dark spot or area of lower illuminance) and annularly shaped heating spots (dashed lines) with two different sizes of area of lower or no irradiation at the center of the heating spots. The temperature increase is shown as distance from the center of the heating spot.

The spot diameters of Fig. 2A are 3.3 mm, with the annular spots having areas of substantially no irradiance (dark spots) with diameter of 0.5 mm and 1 mm. Fig. 2B shows 4 mm diameter heating spots and a heating spot with irradiance profile with a 0.5 mm diameter area of zero irradiance at the center (with abrupt change in illuminance) and one heating spot with irradiance profile such that laser power rises linearly from a relative value of 0.7 to 1 when moving from the center of the spot to the edge of the spot. The irradiance falls to zero when the distance from the center is greater than 2 mm, i.e. the spot diameter is 4 mm.

Fig. 2 shows that with the annularly shaped heating spots, the temperature increase at the area of the retina that is being heated is more even. With the annular spots, the higher temperature peak at the center of the heating spot (or heating area) that is exhibited with the heating beam with a circular heating spot may be avoided. The irradiance profile at the retina may then be more uniform using a heating spot with one or more areas of lower irradiation. A more uniform heat distribution that may be provided with the heating spot comprising an area of lower illumination may be beneficial to deliver a more uniform thermal dose to different parts of the treated area, i.e. the target area of the retina. Fig. 2B shows that with a gradual, e.g. linear increase of the irradiance from the center of the heating spot towards the edges, the temperature elevation at the tissue may be even more even than with other annularly shaped heating spots according to the invention. In some cases, a more uniform temperature profile could be desired, while in other cases it may be advantageous to obtain a temperature profile at the retina where the temperature at the center of the heating area is lower than at the edges. This could be utilized e.g. when the heating treatment is focused at the fovea to ensure that the enter of the fovea is not damaged due to excessively high temperatures.

Differing sizes of smaller circle defining the area of lower or no irradiance could be used to obtain a desired temperature profile in a specific use case. For instance, the smaller circle may have a diameter that is about 5-50% preferably 10-20%, of the diameter of the heating spot. An area with lower irradiance may e.g. be substantially defined by a circle with a diameter from 0.1-1 mm.

The change in irradiance could be realized as occurring gradually, such that there occurs a gradient in irradiance around an area that may be considered to define the smaller circle of the annular shape. Advantageously, the irradiance may increase essentially linearly between a first, lower irradiance value at the center of the heating spot and a second, higher irradiance value at the edge of the heating spot.

The irradiance may rise linearly between the lowest or first value at the center of the spot to a higher, preferably highest, second value at the periphery/edge. This may produce a more uniform temperature profile at the fundus compared to a uniform irradiance profile. Advantageously the irradiance may be configured to be 5-100% higher at the periphery compared to the center, and more advantageously 20-50% higher at the periphery compared to the center.

A heating spot diameter may be selected based on the treated pathology, and may for example be 4 mm. The irradiance value for a 4 mm diameter heating (laser) spot may be 1-10 W/cm² at the center of the heating spot.

As the temperature elevation of retinal tissue caused by heating, such as laser exposure may vary widely between patients, it may be beneficial to perform individualized heating laser power calibration for each patient, where subtherapeutic heating exposure(s) is/are applied, the resulting temperature elevation of the retinal tissue at the target area is determined (e.g. through ERG methods), and the heating power used in the therapy may be optimized to produce a desired temperature elevation of the retinal tissue based on the temperature elevation(s) and laser power(s) used in the subtherapeutic heating exposures.

Customizing the heating spot to be brighter at the edges compared to the centrum may be realized e.g. through optimization methods. In one embodiment, the irradiance profile may be optimized using finite element method thermal modeling to determine the heat distribution of the heating spot realized by a given irradiance profile.

A set of different irradiance profiles may be tested, and the irradiance profile providing a desired heat distribution at the target area may be selected. The lateral temperature profile at the target/heated area is affected by physiological parameters, such as the rate of choroidal perfusion. An irradiance profile may be selected to produce an optimally uniform temperature profile in computational models with naturally occurring range of physiological parameters.

A method of providing a selected or desired irradiance profile may involve computer-implemented simulations or calculations, such as via the aforementioned finite element method thermal modeling, to determine an optimized or selected irradiance profile of the heating beam that fulfils predetermined criteria, such as provides a selected uniformity of heating profile of the heating spot.

In one embodiment, a calibration procedure may be used to determine the power of the heating that is to be used for retinal heating at a therapeutic level, keeping the shape of the irradiance profile constant between patients. Yet, in some embodiments the heating power and/or shape of the irradiance profile may be optimized for the treated patient and retinal area based on a calibration protocol to optimize the irradiance profile of the heating spot.

A calibration protocol may comprise determining retinal temperature from two retinal areas, which may be the center of the target area and an annular ring covering the periphery of the target area. The increase of heating power between the center and the periphery may be decided based on the determined temperature at the center of the treated area and the annular edge of the treated area.

In an exemplary embodiment, the desired irradiance profile may be achieved by illuminating a digital micromirror device with a uniform laser spot and projecting an image of the digital micromirror device onto the retina through a fundus lens. The irradiance profile may be encoded on the digital micromirror device by adjusting the relative time each micromirror spends in an off- and on-state.

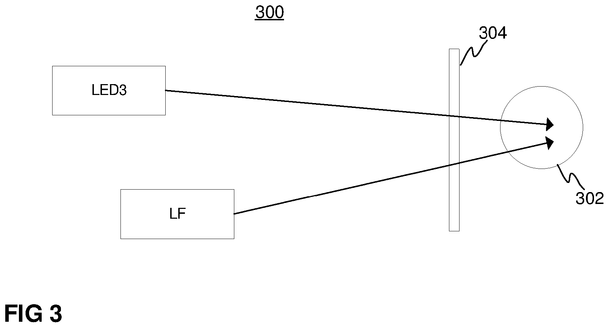

Figure 3 shows one exemplary device 300 for heating a target area of the retina of an eye 302. The device 300 comprises at least a heating light source LF for providing a heating beam to heat at least the target area, wherein the heating beam comprises an irradiance profile providing a heating spot comprising an area of lower irradiance.

The device 300 may also comprise a stimulus light source LED3 configured to provide a stimulus beam. The stimulus beam may be used to illuminate at least an area from which ERG signals are to be obtained, which may be equivalent to the target area which is heated/to be heated during the retinal heating. The device may also comprise optical elements 304, which may correspond to the optical elements described elsewhere herein.

In one embodiment, the irradiance profile of the heating spot may be controlled by e.g. a mask corresponding to M1 of Fig 1, where M1 may be a graded neutral density filter that passes the desired irradiance profile, or a digital micromirror device, which reflects the desired irradiance profile

The invention has been explained above with reference to the aforementioned embodiments, and several advantages of the invention have been demonstrated.

## Claims

1. A device for obtaining retinal electroretinography signals from a target area of the retina, the device comprising means for obtaining an electrical response signal from the target area and a heating system for heating at least the target area, wherein the heating system comprises a heating light source (LF) for providing a heating beam to heat at least the target area, the device additionally comprising at least one light source (LED1, LED2, LED3) configured to provide
- a stimulus beam configured to illuminate the target area for inducing an electroretinography signal and
- a light adapting background beam configured to illuminate the retina at least in an area outside of the target area,
**characterized in that** the light adapting background beam is provided for light adapting the area outside of the target area and suppressing electroretinography signaling therefrom and **in that** the device is further adapted to provide a central background light beam configured to illuminate at least the target area of the retina and to maintain a light adaptation level of the target area.

2. The device of claim 1, wherein the light adapting background beam is adapted to illuminate an area on the fundus from essentially at or near an outer perimeter of the target area to or beyond the equator of the eye.

3. The device of any previous claim, wherein the device comprises means for preventing the light adapting background beam from reaching a fundus imaging system.

4. The device of claim 3, wherein the device comprises an optical notch filter (F2) for blocking the light adapting background beam from fundus imaging.

5. The device of claim 3, wherein the light adapting background beam comprises polarized light and the light adapting background beam is blocked from fundus imaging using a polarizer.

6. The device of claim 3, wherein the device comprises means for modulating the light adapting background beam with an on/off waveform and the device comprises an imaging module (IM1, IM2) comprising a camera sensor which is synchronized to be exposed essentially only when the light adapting background beam is off.

7. The device of any previous claim, wherein the light adapting background beam comprises an area of lower or no illumination corresponding to the stimulus beam such that when the stimulus beam and the light adapting background beam are directed at a final location with respect to an eye, the area of lower or no illumination essentially overlaps with the stimulus beam.

8. The device of any previous claim, wherein the heating beam has an essentially circular, substantially homogenous irradiance profile and a diameter of 1-6 mm at the fundus.

9. The device of claim 8, wherein the stimulus beam is of equal size or smaller than the heating beam used for heating at least the target area.

10. The device of any of claims 8-9, wherein the heating light source is configured to provide an aiming beam having essentially equivalent beam size and irradiance profile as the heating beam, optionally further wherein a power of the aiming beam is configured to be reduced when the heating beam is turned on to maintain an essentially steady illuminance at the target area.

11. The device of any previous claim, wherein the brightness of the central background light beam is configured to reduce as a heating system is turned on to maintain a steady illuminance at the target area.

12. The device of any previous claim, wherein the central background light beam has a brightness of over 50 lux, advantageously over 100 lux.

## Patentansprüche

1. Vorrichtung zum Erhalten von retinalen Elektroretinographiesignalen aus einem Zielbereich der Netzhaut, wobei die Vorrichtung Mittel zum Erhalten eines elektrischen Antwortsignals aus dem Zielbereich und ein Heizsystem zum Erhitzen von zumindest dem Zielbereich umfasst, wobei das Heizsystem eine Heizlichtquelle (LF) zum Bereitstellen eines Heizstrahls zum Erhitzen von zumindest dem Zielbereich umfasst, wobei die Vorrichtung zusätzlich mindestens eine Lichtquelle (LED1, LED2, LEDS) umfasst, die dazu konfiguriert ist, Folgendes bereitzustellen:
- einen Stimulus-Strahl, der konfiguriert ist, um den Zielbereich zu beleuchten, um ein Elektroretinographiesignal zu induzieren, und
- einen lichtanpassenden Hintergrundstrahl, der konfiguriert ist, um die Netzhaut zumindest in einem Bereich außerhalb des Zielbereichs zu beleuchten,
**dadurch gekennzeichnet, dass** der lichtanpassende Hintergrundstrahl zum Lichtanpassen des Bereichs außerhalb des Zielbereichs und zur Unterdrückung der Elektroretinographiesignalgebung davon vorgesehen ist, und dass die Vorrichtung ferner dazu geeignet ist, einen zentralen Hintergrundlichtstrahl bereitzustellen, der dazu konfiguriert ist, zumindest den Zielbereich der Netzhaut zu beleuchten und einen Lichtanpassungspegel des Zielbereichs aufrechtzuerhalten.

2. Vorrichtung nach Anspruch 1, wobei der lichtanpassende Hintergrundstrahl dazu geeignet ist, einen Bereich auf dem Augenhintergrund von im Wesentlichen an oder nahe einem äußeren Umfang des Zielbereichs bis oder über den Äquator des Auges hinaus zu beleuchten.

3. Vorrichtung nach einem der vorhergehenden Ansprüche, wobei die Vorrichtung Mittel umfasst, die verhindern, dass der lichtanpassende Hintergrundstrahl ein Augenhintergrund-Bildgebungssystem erreicht.

4. Vorrichtung nach Anspruch 3, wobei die Vorrichtung einen optischen Sperrfilter (F2) umfasst, der den lichtanpassenden Hintergrundstrahl von der Augenhintergrund-Bildgebung abhält.

5. Vorrichtung nach Anspruch 3, wobei der lichtanpassende Hintergrundstrahl polarisiertes Licht umfasst und der lichtanpassende Hintergrundstrahl durch einen Polarisator von der Fundusabbildung blockiert wird.

6. Vorrichtung nach Anspruch 3, wobei die Vorrichtung Mittel zum Modulieren des lichtanpassenden Hintergrundstrahls mit einer Ein/Aus-Wellenform umfasst und die Vorrichtung ein Abbildungsmodul (IM1, IM2) umfasst, das einen Kamerasensor umfasst, der so synchronisiert ist, dass er im Wesentlichen nur dann belichtet wird, wenn der lichtanpassende Hintergrundstrahl ausgeschaltet ist.

7. Vorrichtung nach einem der vorhergehenden Ansprüche, wobei der lichtanpassende Hintergrundstrahl einen Bereich geringerer oder keiner Beleuchtung aufweist, der dem Stimulusstrahl entspricht, so dass, wenn der Stimulusstrahl und der lichtanpassende Hintergrundstrahl auf eine Endposition in Bezug auf ein Auge gerichtet sind, der Bereich geringerer oder keiner Beleuchtung im Wesentlichen mit dem Stimulusstrahl überlappt.

8. Vorrichtung nach einem der vorhergehenden Ansprüche, wobei der Heizstrahl ein im Wesentlichen kreisförmiges, im Wesentlichen homogenes Bestrahlungsprofil und einen Durchmesser von 1 bis 6 mm am Augenhintergrund aufweist.

9. Vorrichtung nach Anspruch 8, wobei der Stimulusstrahl gleich groß oder kleiner als der Heizstrahl ist, der zum Erhitzen zumindest des Zielbereichs verwendet wird.

10. Vorrichtung nach einem der Ansprüche 8 bis 9, wobei die Heizlichtquelle so konfiguriert ist, dass sie einen Zielstrahl mit im Wesentlichen gleicher Strahlgröße und gleichem Bestrahlungsprofil wie der Heizstrahl liefert, wobei optional weiterhin eine Leistung des Zielstrahls so konfiguriert ist, dass sie reduziert wird, wenn der Heizstrahl eingeschaltet wird, um eine im Wesentlichen konstante Beleuchtungsstärke im Zielbereich aufrechtzuerhalten.

11. Vorrichtung nach einem der vorhergehenden Ansprüche, wobei die Helligkeit des zentralen Hintergrundlichtstrahls so konfiguriert ist, dass sie reduziert wird, wenn ein Heizsystem eingeschaltet wird, um eine konstante Beleuchtungsstärke im Zielbereich aufrechtzuerhalten.

12. Vorrichtung nach einem der vorhergehenden Ansprüche, wobei der zentrale Hintergrundlichtstrahl eine Helligkeit von über 50 Lux, vorteilhafterweise über 100 Lux aufweist.

## Revendications

1. Dispositif permettant d'obtenir des signaux d'électrorétinographie rétinienne provenant d'une zone cible de la rétine, le dispositif comprenant un moyen pour obtenir un signal de réponse électrique provenant de la zone cible et un système de chauffage pour chauffer au moins la zone cible, dans lequel le système de chauffage comprend une source de lumière chauffante (LF) pour fournir un faisceau chauffant pour chauffer au moins la zone cible, le dispositif comprenant en outre au moins une source de lumière (LED1, LED2, LED3) configurée pour fournir
- un faisceau de stimulus configuré pour éclairer la zone cible afin d'induire un signal d'électrorétinographie et
- un faisceau d'arrière-plan d'adaptation de lumière configuré pour éclairer la rétine au moins dans une zone à l'extérieur de la zone cible,
**caractérisé en ce que** le faisceau d'arrière-plan d'adaptation de lumière est prévu pour adapter la lumière de la zone à l'extérieur de la zone cible et supprimer la signalisation d'électrorétinographie à partir de celle-ci et **en ce que** le dispositif est en outre conçu pour fournir un faisceau lumineux d'arrière-plan central configuré pour éclairer au moins la zone cible de la rétine et afin de maintenir un niveau d'adaptation de lumière de la zone cible.

2. Dispositif selon la revendication 1, dans lequel le faisceau d'arrière-plan d'adaptation de lumière est conçu pour éclairer une zone sur le fond d'œil essentiellement depuis ou à proximité d'un périmètre externe de la zone cible jusqu'à ou au-delà de l'équateur de l'œil.

3. Dispositif selon une quelconque revendication précédente, dans lequel le dispositif comprend un moyen pour empêcher le faisceau d'arrière-plan d'adaptation de lumière d'atteindre un système d'imagerie de fond d'œil.

4. Dispositif selon la revendication 3, dans lequel le dispositif comprend un filtre coupe-bande optique (F2) pour bloquer le faisceau d'arrière-plan d'adaptation de lumière à partir de l'imagerie de fond d'œil.

5. Dispositif selon la revendication 3, dans lequel le faisceau d'arrière-plan d'adaptation de lumière comprend une lumière polarisée et le faisceau d'arrière-plan d'adaptation de lumière est bloqué à partir de l'imagerie de fond d'œil à l'aide d'un polariseur.

6. Dispositif selon la revendication 3, dans lequel le dispositif comprend un moyen pour moduler le faisceau d'arrière-plan d'adaptation de lumière avec une forme d'onde marche/arrêt et le dispositif comprend un module d'imagerie (IM1, IM2) comprenant un capteur de caméra qui est synchronisé pour être exposé essentiellement uniquement lorsque le faisceau d'arrière-plan d'adaptation de lumière est éteint.

7. Dispositif selon une quelconque revendication précédente, dans lequel le faisceau d'arrière-plan d'adaptation de lumière comprend une zone d'éclairage inférieur ou nul correspondant au faisceau de stimulus de sorte que lorsque le faisceau de stimulus et le faisceau d'arrière-plan d'adaptation de lumière sont dirigés vers un emplacement final par rapport à un œil, la zone d'éclairage inférieur ou nul chevauche essentiellement le faisceau de stimulus.

8. Dispositif selon une quelconque revendication précédente, dans lequel le faisceau chauffant a un profil d'irradiance essentiellement circulaire et sensiblement homogène et un diamètre de 1 à 6 mm au niveau du fond d'œil.

9. Dispositif selon la revendication 8, dans lequel le faisceau de stimulus est de taille égale ou inférieure au faisceau chauffant utilisé pour chauffer au moins la zone cible.

10. Dispositif selon l'une quelconque des revendications 8 et 9, dans lequel la source de lumière chauffante est configurée pour fournir un faisceau de visée ayant une taille de faisceau et un profil d'irradiance essentiellement équivalents à ceux du faisceau chauffant, éventuellement en outre dans lequel une puissance du faisceau de visée est configurée pour être réduite lorsque le faisceau chauffant est allumé afin de maintenir un éclairement essentiellement stable au niveau de la zone cible.

11. Dispositif selon une quelconque revendication précédente, dans lequel la luminosité du faisceau lumineux d'arrière-plan central est configurée pour diminuer lorsqu'un système de chauffage est allumé afin de maintenir un éclairement stable au niveau de la zone cible.

12. Dispositif selon une quelconque revendication précédente, dans lequel le faisceau lumineux d'arrière-plan central a une luminosité supérieure à 50 lux, avantageusement supérieure à 100 lux.
